Europäisches Patentamt

European Patent Office

Office européen des brevets

(11) Veröffentlichungsnummer: **0 394 997**
**A1**

(12) **EUROPÄISCHE PATENTANMELDUNG**

(21) Anmeldenummer: 90107832.9

(22) Anmeldetag: 25.04.90

(51) Int. Cl.⁵: **G01N 27/30, C12M 1/40**

(30) Priorität: 26.04.89 DE 3913815

(43) Veröffentlichungstag der Anmeldung:
31.10.90 Patentblatt 90/44

(84) Benannte Vertragsstaaten:
**AT BE CH DE DK ES FR GB IT LI NL SE**

(71) Anmelder: **EUROPÄISCHES LABORATORIUM FÜR MOLEKULARBIOLOGIE (EMBL)**
**Meyerhofstrasse 1**
**D-6900 Heidelberg 1(DE)**

(72) Erfinder: **Pattus, Franc, Dr.**
**Mühlwingertweg 3**
**D-6913 Mühlhausen(DE)**

(74) Vertreter: **Huber, Bernhard, Dipl.-Chem. et al.**
**Möhlstrasse 22 Postfach 860 820**
**D-8000 München 86(DE)**

(54) **Porine enthaltende biospezifische Membranen und ihre Verwendung.**

(57) Es werden Membranen beschrieben, die aus einer Matrix mit darin eingelagerten Porinen gram negativer Bakterien bestehen. Diese substrat-spezifischen Membranen eignen sich hervorragend für die Verwendung als Membranen für oberflächen-selektive Biosensoren. Es wird auch ein Verfahren zur Bestimmung von organischen Substanzen durch Messung der durch diese Substanzen hervorgerufenen Veränderung der Ionendurchlässigkeit einer Membran beschrieben, wobei man als Membran eine ein für die zu bestimmende organische Substanz spezifisches Porin gram negativer Bakterien enthaltende Membran einsetzt.

EP 0 394 997 A1

## Porine enthaltende biospezifische Membranen und ihre Verwendung

Die Erfindung betrifft Porine gram negativer Bakterien enthaltende biospezifische Membranen und ihre Verwendung als Membranen von Biosensoren.

Biospezifischen Sensoren kommt in der Analytik, insbesondere für medizinisch-diagnostische und Lebensmitteluntersuchungen, aber auch zur Untersuchung von Reaktionsabläufen, eine ständig wachsende Bedeutung zu.

Das Arbeitsprinzip von biospezifischen Sensoren beruht im wesentlichen auf drei Schritten: (1) die Erfassung des zu bestimmenden Moleküls über seine spezifische Beeinflussung des sensierenden Systems, (2) eine dadurch hervorgerufene bestimmbare Störung des sensierenden Systems, z.B. des Membrangleichgewichtes einer Sensormembran, und (3) die Konvertierung dieser Störung in eine meßbare Größe. Bei oberflächen-selektiven Sensoren erfolgt die Sensierung über einen Mechanismus an der Oberfläche des Sensors, z.B. durch eine biospezifische Bindung der Moleküle an die Sensoroberfläche.

Eines der wichtigsten Kriterien für die praktische Anwendung von biospezifischen Oberflächen-Sensoren ist dabei die Bereitstellung einer geeigneten Sensoroberfläche, die bestimmte Anforderungen erfüllen muß, wie z.B. hohe Spezifität, mechanische Stabilität und Regenerierbarkeit (W.M. Reichert et al, Thin Solid Films, 152 (1987) 345-367).

Ein Beispiel für Sensoroberflächen sind Membranen mit darin eingebetteten Rezeptoren, die nach einem Rezeptor-Transmitter-Mechanismus arbeiten, wobei durch die Bindung eines Transmitter-Moleküls (T) an den Rezeptor (R) eine Änderung der Struktur erfolgt, wodurch z.B. Ionenkanäle durch die Membran (trans membrane ion channels) geöffnet werden, und dadurch der Ionenfluß durch die Membran und damit deren Leitfähigkeit verändert werden. Obwohl solche Sensoroberflächen eine beachtliche Effizienz aufweisen, weil bereits ein oder zwei an einem Rezeptor gebundene Moleküle eine signifikante Veränderung der Membraneigenschaften, z.B. der Leitfähigkeit, hervorrufen können, haben sie auf Grund experimenteller Schwierigkeiten bei ihrem Aufbau in der Praxis bisher keine Verwendung gefunden (vgl. W.M. Reichert et al, l.c. Seite 352).

Aufgabe der vorliegenden Erfindung war deshalb die Bereitstellung von für biospezifische Oberflächensensoren geeigneten Membranen mit hoher Effizienz, die sich leicht und reproduzierbar aufbauen lassen und sich auf Grund ihrer Eigenschaften gut für Oberflächensensoren eignen.

Es wurde gefunden, daß sich diese Aufgabe durch Verwendung einer anderen Klasse von Ionenkanäle enthaltenden Membranen (ion channel membranes) lösen läßt, nämlich durch die Verwendung von Membranen, die aus einer Matrix mit darin eingelagerten Porinen aus gram negativen Bakterien bestehen.

Es ist bekannt, daß Porine, wie z.B. Phosphoporin (PhoE),ein Protein der äußeren Zellmembran von E. coli, wenn sie in Lipid-Doppelschichten inkorporiert werden, Kanäle bilden, die mit denen im Matrix-Porin vergleichbar sind. Ein Phosphoporin-Trimer enthält 3 Poren (Kanäle), durch die Ionen bis zu einem Molekulargewicht von ca. 500 Dalton hindurchdiffundieren können. Spezifische höhermolekulare organische Substanzen werden an ein spezifisches Porin gebunden, und inhibieren den Ionendurchfluß durch die Kanäle (vgl. B. Dargent et al, EMBO J. 5 (1986) 773-778, B. Dargent et al, FEBS Let. 220 (1987) 136-142 und C. Maier et al, J. Biol. Chem. 263 (1988) 2493-2499). Porine können also ein für sie spezifisches Substrat erkennen, und nach Bindung an das Protein inhibiert dieses Substrat die Ionenleitung durch die Kanäle, also die elektrische Durchlässigkeit der die Porine enthaltenden Membran. Die Bindungskonstanten der organischen Substanzen an die Porine sind in der Regel unabhängig von der Ionenzusammensetzung des wäßrigen Mediums (wäßrige Ionenlösung), und die Porine bilden vergleichsweise große, mit Wasser ausgefüllte Kanäle, deren Leitwert zur spezifischen Leitfähigkeit des wäßrigen Mediums proportional ist. Durch Messung der Leitfähigkeit der Lösung und des Ionenstroms durch die Membran lassen sich deshalb die den Ionendurchfluß inhibierenden organischen Substanzen, z.B. Maltose oder Maltodextrine bei Anwendung des dafür spezifischen Porins Maltoporin von E. coli, bestimmen.

Gegenstand der vorliegenden Erfindung ist deshalb die Verwendung von Porinen gram negativer Bakterien für biospezifische Membranen von Biosensoren.

Unter den erfindungsgemäß verwendeten Porinen sind vor- und nachstehend auch geeignete porin-ähnliche Proteine der äußeren Zellmembran gram negativer Bakterien und Mutanten der Porine zu verstehen; mit solchen Mutanten, die durch konventionelle Mutagenese oder gentechnologische Methoden erhältlich sind (vgl. z.B. A. Clune et al, Biochem. Biophys. Res. Com. 121 (1984) 34-40 und B. Dargent et al, J. Mol. Biol. 201 (1988) 497-506) läßt sich gegebenenfalls z.B. eine Steigerung der Selektivität und/oder Affinität gegenüber den zu bestimmenden Substanzen erreichen.

Die biospezifische Membran besteht aus einer Matrix, in der die Porine eingelagert sind. Als Matrixmaterial können für Zwei-Schichten-Membranen (bilayer membranes, BLM) übliche Lipide, vorzugsweise Phospholipide, eingesetzt werden (vgl. W.M. Reichert et al, lc., EMBO J. 5 (1986) 773-778), oder auch z.B. vernetztes Albumin oder ein gegebenenfalls vernetztes synthetisches Polymeres, wie z.B. Polyacrylamid, Triacetylcellulose, PMMA, oder insbesondere Polyvinylchlorid (PVC).

Die Herstellung der erfindungsgemäß verwendeten Membranen kann nach einer der zur Immobilisierung oder Rekonstitution von Proteinen in einer Matrix bekannten Methode (vgl. z.B. H. Freiser, ed. (1978) Ion-selective electrodes in Analytical Chemistry, Vol. 1, Plenum Press, H. Freiser, ed. (1980) Ion-selective electrodes in Analytical Chemistry, Vol. 2, Plenum Press, J. Koryta (1975) Ion-selective electrodes, Cambridge University Press, S.A. Barker, Immobilization of the biological components of biosensors, S. 85-99 In Biosensors, fundamental and applications (1987) A. Turner, I. Karube and G. Wilson Eds. Oxford University Press, London, M. Coughlan et al, J. Microbiol. Methods 8 (1988) 1-50 und D.L. Dorset et al, J. Mol. Biol. 165 (1983) 701-710) bzw. auf eine zur Herstellung von BLM übliche Weise erfolgen, z.B. durch die bei W.M. Reichert et al, lc. beschriebenen Techniken, oder wie bei B. Dargent et al, EMBO J. 5 (1986) 773-778, beschrieben. Daneben kann auch noch das Verfahren der "self-assembly" von Monoschichten in Frage kommen (vgl. z.B. B. Dargent et al, J. Mol. Biol. 201 (1988) 497-506).

Unter Verwendung eines synthetischen Polymers, wie z.B. von Polyvinylchlorid, als Matrix, können die Membranen hergestellt werden, indem man die Herstellung des Polymers in Gegenwart von Porinen durchführt, d.h. die Porine dem Polymerisationsansatz vor der Polymerisation zusetzt.

Vorzugsweise enthalten die biospezifischen Membranen das Porin in einem Verhältnis von Porin/Matrix von $10^{-1}$ bis $10^{-4}$ (w/w).

Erfindungsgemäß verwendete Porine gram negativer Bakterien sind z.B. die folgenden (vgl. R. Benz und K. Bauer, Eur.J.Biochem. 176 (1988) 1-19):

3

| PORIN | BAKTERIENSPECIES |
|---|---|
| OmpF | *Escherichia coli* |
| OmpC | |
| Lc | |
| Lc (HK 253) | |
| NmpC | |
| K | |
| OmpF | *Salmonella* |
| | *typhimurium* |
| OmpC | typhimurium |
| OmpD | |
| PhoE | |
| 37-kDA | *Enterobacter cloacae* |
| 40-kDa | |
| PhoE | |
| PhoE | *K. pneumoniae* |
| I | *N. gonorrhoae* |
| F | *P. aeruginosa* |
| E | *Y. pestis* |
| MOMP | *L. pneumophila* |
| 43-kDa | *C. jejuni* |
| 46-kDa | *C. fetus* |
| 32-kDa | *S. aurantia* |
| 42-kDa | *A. salmonicida* |
| 40-kDa | *P. mirabilis* |
| 40-kDa | *H. influenza* |
| Group 2 | *B. abortus* |
| 33-kDa | *P. denitrificans* |
| 43-kDa | *R. capsulatus* |
| 47-kDa | *R. sphaeroides* |
| Porin | *A. variabilis* |
| LamB | *E.coli* |
| Tsx | *E.coli* |
| DI | *P. aeruginosa* |
| P | *P. aeruginosa* |
| LamB | *S. typhimurium* |

und insbesondere LamB (Maltoporin, B. Dargent et al, FEBS Let. 220 (1987) 136-142), PhoE (Phosphoporin, B. Dargent et al, EMBO J., 5 (1986) 773-778) und Tsx (C. Maier et al, J. Biol. Chem. 263 (1988) 2493-2499).

Gegenstand der Erfindung ist deshalb auch die Verwendung einer wie oben beschriebenen Membran aus einer Matrix mit darin ein gelagerten Porinen gram negativer Bakterien als biospezifische Membran von Biosensoren.

Ein weiterer Gegenstand der Erfindung sind auch neue biospezifische Membranen zur Verwendung als sensitive Membran in Biosensoren, bestehend aus einer Matrix aus einem gegebenenfalls vernetzten synthetischen Polymeren, wie z.B. Polyacrylamid, Triacetylcellulose, PMMA, und insbesondere Polyvinylchlorid (PVC), und darin eingelagerten Porinen gram negativer Bakterien, insbesondere mit den vorstehend bevorzugt genannten Porinen.

Ein weiterer Gegenstand ist auch ein Verfahren zur Bestimmung von organischen Substanzen durch Messung der durch diese Substanzen hervorgerufenen Veränderung der Ionendurchlässigkeit einer Membran, das dadurch gekennzeichnet ist, daß man als Membran eine ein für die zu bestimmende organische Substanz spezifisches Porin gram negativer Bakterien enthaltende Membran einsetzt und die durch die zu bestimmende organische Substanz hervorgerufene Veränderung der Durchlässigkeit von Ionen, die unter der Einwirkung eines elektrischen Feldes durch die Membran hindurchtreten, bestimmt.

Zur Bestimmung der Veränderung der Ionendurchlässigkeit kann mit jeder geeigneten Methoden (Meßprinzip) und Einrichtung gearbeitet werden, wie sie für biospezifische oberflächen-selektive Sensoren unter Verwendung von Membranen üblich ist, wobei als Membran eine erfindungsgemäße Membran verwendet wird, also z.B. mit amperometrischen, potentiometrischen, konduktometrischen Methoden und Anordnungen, aber auch z.B. mit optischen und anderen für derartige Anwendungen bekannten Methoden (vgl. W.M. Reichert et al, lc.). Vorzugsweise wird die Veränderung der Durchlässigkeit konduktometrisch bestimmt als Differenz der Leitfähigkeiten durch die Membran vor und nach ihrem Kontakt mit den zu bestimmenden organischen Substanzen.

Die Messungen werden z.B. auf der Basis eines (1) Biosensors mit Enzymelektrode (vgl. US-PS 3 359 355; M. Coughlan et al, J. Microbiol. Methods 8 (1988) 1-50; J.H.T. Luong et al, TIBTECH-DECEMBER 1988 (Vol. 6) 310-316), (2) eines mikroelektronischen konduktometrischen Biosensors mit Enzymelektrode (L. Clark jr., The enzyme electrode In Biosensors, fundamental and applications (1987) A. Turner, I. Karube und G. Wilson Eds., S. 3-12, Oxford University Press, London), (3) eines nach dem INF[ET]-System (L.D. Watson et al, Biosensors, 3 (1987) 10-115) arbeitenden Biosensors, und insbesondere (4) eines Biosensors mit auf einem Träger befindlichen planaren Zwei-Schichten-Membranen (supported planar bilayer membranes) (vgl. G. Blackburn, Chemically sensitive field effect transistors, S. 481-530. In Biosensors, fundamental and applications (1987) A. Turner, I. Karube and G. Wilson Eds. Oxford University Press, London; H. Ringsdorf et al, Thin Solid Films 152 (1987) 207-222 und K. Hongyo et al, Langmuir, 1987, 3:827-830) durchgeführt.

Das Prinzip des Biosensors 1 beruht auf einer semipermeablen dünnen Membran, die das Porin, z.B. Maltoporin, in hoher Dichte enthält. In diesem Fall wird nur ein Teil der Leitfähigkeit der Membran durch die zu bestimmende Substanz (das Substrat) beeinflußt. Die Leitfähigkeit wird sowohl durch die Diffusion durch die semipermeable Membran (aspezifische Leitfähigkeit) als auch durch die großen Öffnungen der Porine (3 x 1.5 nm Durchmesser/Trimer) verursacht, wobei der durch die Öffnungen der Porine verursachte Anteil der Leitfähigkeit durch das Substrat (z.B. Dextrine) inhibiert wird. Wegen der hohen Dichte der Porine innerhalb der Membranmatrix und den großen Kanälen stellt die gegenüber den Maltodextrinen sensitive Leitfähigkeit einen wesentlichen Teil der gesamten Leitfähigkeit des Biosensors dar. Die verwendeten Protein-Gel-Membranen werden typischerweise gebildet durch Immobilisierung des Proteins (Porins) in einer Matrix aus vernetztem Albumin, Polyacrylamid oder Triacetylcellulose.

Das Prinzip des Biosensors 2 ist im wesentlichen identisch mit dem von 1; der Unterschied liegt nur darin, daß die Veränderung der Leitfähigkeit bestimmt wird mit einer Anordnung, in der das immobilisierte Protein zu einer planaren mikroelektronischen Leitfähigkeitszelle mit definierter Geometrie proximal angeordnet ist.

Ein weiterer Vorteil der erfindungsgemäß verwendeten Membranen und des Verfahrens ist es, daß es sich bei den Porinen um extrem stabile Proteine handelt. Sie bilden Trimere, die nur beim Erhitzen auf ca. 95°C in Gegenwart von Natriumdodecylsulfat (SDS) dissoziieren. Die Substratbindung ist reversibel und die Porine sind sehr substratspezifisch.

Die Bestimmung der Substrate kann quantitativ erfolgen, wobei aber zu berücksichtigen ist, daß die Porine nahe verwandte chemische Substanzen, die sich z.B. nur durch ihre Kettenlänge unterscheiden, also Homologe mit ähnlichem Molekulargewicht darstellen (z.B. Maltodextrine unterschiedlicher Kettenlänge) nicht unterscheiden können, und deshalb nur die Summe derartiger Substanzen quantitativ erfaßbar ist.

Am Beispiel von Maltoporin wurde die Porin-Selektivität gegenüber natürlichen und synthetischen Zuckern sowohl in vivo als auch in vitro ausführlich untersucht (vgl. H. Heine et al, Gene, 53 (1987) 287-292; M. Luckey und H. Nikaido, Proc. Natl. Acad. Sci. USA 77 (1980) 167-171; T. Ferenci et al, Biochim. Biophys. Acta. 860 (1986) 44-50; und R. Benz et al, J. Membrane Biol. 100, 21-29 (1987). Aus diesen Untersuchungen kann der Schluß gezogen werden, daß Maltoporin, beispielhaft für andere Porine, eine sehr hohe Selektivität zeigt; dabei muß zwischen Ergebnissen, die mittels Durchflußmessungen erhalten werden, und solchen, die mittels Leitfähigkeitsmessungen (Inhibierung der Leitfähigkeit) erhalten werden, differenziert werden. Glucose oder Lactose diffundieren z.B. auf Grund der geringen Molekülgröße recht gut durch die Porinkanäle, und beeinflussen die Leitfähigkeit der Kanäle nicht, weil sie vom Porinprotein nicht erkannt werden. So werden z.B. die von Maltoporin, das in eine planare Lipid-Doppelschicht eingebaut ist, gebildeten Kanäle durch Maltose und Maltodextrine inhibiert, nicht aber z.B. durch das ähnlich aufgebaute Disacharid Lactose mit einem deutlich verschiedenen Molekulargewicht.

Die Messungen sind von der in dem wäßrigen Medium verwendeten Ionenart unabhängig, weil die Leitfähigkeit der Porinkanäle (z.B. von Maltoporin) zur Leitfähigkeit des wäßrigen Mediums streng proportional ist. Um die Reaktion des Biosensors für den Fall der Abwesenheit der zu bestimmenden Substanz (z.B. Maltose oder Maltodextrine) zu bestimmen, kann deshalb eine gleichzeitige Messung der Leitfähigkeit, z.B. mit einem klassischen Konduktometer, verwendet werden. Eine Alternative dazu ist die Verwendung von zwei Biosensoren, wobei einer das substrat-spezifische Porin (z.B. Maltoporin) enthält, der andere ein für die Substanz aspezifisches Porin (z.B. Ompf oder Ompc-Protein); dieses alternative Verfahren besitzt den Vorteil, daß man auf diese Weise Störungen der Messung durch andere Verbindungen ausschalten kann.

Die meßbare Konzentration für die zu bestimmende Substanz, z.B. Maltose , liegt im allgemeinen im Bereich von 5 x $10^{-6}$ bis $10^{-2}$ Mol/Liter. Dieser Konzentrationsbereich kann durch geeignete Mutanten mit höherer Affinität gegenüber den zu bestimmenden Substanzen nach unten hin noch erweitert werden (vgl. z.B. A. Clune et al, Biochem. Biophys. Res. Com. 121 (1984) 34-40).

Für quantitative Messungen kann die Kurve $G_{max}/(G_{max}-G_c)$ als Funktion von 1/c dienen, die eine gerade Linie ergibt. Es bedeuten: $G_{max}$ = Leitfähigkeit in Abwesenheit der zu bestimmenden Substanz (z.B. von Maltodextrinen); $G_c$ = Leitfähigkeit in Anwesenheit der zu bestimmenden Substanz (z.B. von Maltodextrinen) mit der Konzentration c .

Bei der quantitativen Auswertung ist aber zu berücksichtigen, daß bei der Bestimmung Mischungen von Homologen mit ähnlicher Molekülgröße (z.B. von Maltodextrinen verschiedener Länge) erfaßt werden.

Die Porine können in beträchtlichen und praktisch brauchbaren Mengen aus den die Porine produzierenden Bakterienstämmen hergestellt werden, die nur einen Porintypus produzieren (vgl. z.B. J.M. Neuhaus et al, EMBO J. 2 (1983) 1987-1991; B. Dargent et al, EMBO J. 5 (1986) 773-778).

Als Ionen des wäßrigen Mediums, deren Durchlässigkeit durch die erfindungsgemäß verwendeten Membranen bestimmt wird, kommen im allgemeinen alle anorganischen oder auch organischen Ionen oder Komplexionen in Frage, deren Molekulargewicht 500 Dalton nicht übersteigt; vorzugsweise werden Alkali- oder Erdalkaliionen, wie z.B. $Ca^{2+}$, $Mg^{2+}$ , Na+, K+, Li+, oder $NH_4^+$-Ionen verwendet. Die wäßrige Lösung enthält vorzugsweise ein übliches Konservierungsmittel, wie z.B. ein Azid, vorzugsweise ein solches mit den gleichen Kationen wie der Elektrolyt. Die Art der Anionen im Elektrolyten richtet sich nach der verwendeten Meßelektrode; bei Verwendung einer Ag/AgCl-Elektrode ist das Anion Chlorid.

In einer Ausführungsform der vorliegenden Erfindung wird als Porin Maltoporin verwendet. Mit einem Maltoporin-Biosensor lassen sich z.B. Konzentrationsbestimmungen von Stärke, Amylopectin, Maltodextrinen und Maltose in Flüssigkeiten durchführen, z.B. in der Nahrungsmittelindustrie, bei Fermentationsprozessen, oder in der klinischen Diagnostik.

In weiteren bevorzugten Ausführungsformen können die folgenden Porine bzw. Porin-Biosensoren verwendet werden: $Ts_x$ zur Bestimmung von Nucleosiden, Porin P aus Pseudomonas aeruginosa zur Phosphatbestimmung, Phosphoporin PhoE zur Polyphosphatbestimmung, und das Vitamin B12-Rezeptor-Porin zur Bestimmung von Vitamin B12.

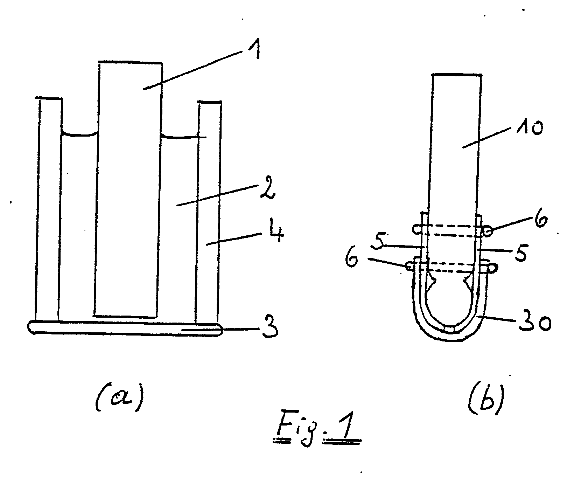

In den Figuren 1a und 1b sind schematisch zwei Ausführungsformen eines erfindungsgemäßen Biosensors unter Verwendung einer Porine enthaltenden Membran dargestellt. Es bedeuten: 1, 10 eine Sensorelektrode; 2 den Elektrolyten; 3, 30 eine erfindungsgemäße Matrix/Porin-Membran; 4 eine für den Elektrolyten und die Probelösung undurchlässige Wand, die Bestandteil des Elektrodenkörpers sein kann.

Figur 1b betrifft eine für eine praktische Anwendung zweckmäßige Ausführungsform eines erfindungsgemäßen Biosensors: Die erfindungsgemäße Matrix/Porin-Membran (30) liegt auf einem Abstandhalter (5) auf, der als Netz aus einem unter den Betriebsbedingungen inerten und nicht-leitenden Material z.B. aus Kunststoff, wie z.B. Polyamid, ausgebildet ist. Der Abstandhalter (5) und die darauf aufliegende Membran (30) werden über geeignete Vorrichtungen, z.B. Hartgummi- oder Kunststoffringe (6) an die Sensorelektrode (10) gedrückt.

Die Biosensoren tauchen mit ihrem Teil, der die Sensormembran enthält, in die zu untersuchende Probelösung ein; als Referenzelektrode, die ebenfalls in die zu untersuchende Probelösung eintaucht, kann eine dafür übliche Elektrode, z.B. Silber/Silberchlorid mit Salzbrücken, verwendet werden.

Es wird die Differenz der Leitfähigkeit durch die Sensormembran gemessen zwischen dem Zustand (1) bei dem der Biosensor in die Elektrolytlösung eintaucht; und (2) bei dem der Biosensor in die zu untersuchende Probelösung eintaucht.

Gegenstand der Erfindung ist auch ein Biosensor, bestehend aus einer Sensorelektrode ( 1, 10 ), die zumindest teilweise über eine semipermeable Membran ( 3, 30 ) in Verbindung mit der zu untersuchenden Probelösung steht, und dadurch gekennzeichnet ist, daß die semipermeable Membran ( 3, 30 ) eine Membran aus einer Matrix und darin eingelagerten Porinen gramnegativer Bakterien ist.

In einer zweckmäßigen Ausführungsform ist die Elektrode ( 1, 10) stabförmig und an ihrem in die Probelösung eintauchenden Ende vorzugsweise abgerundet ausgebildet, wobei auf diesem Ende ein Netz (5) aus einem nichtleitenden Material aufliegt, und die Membran ( 3, 30 ) auf diesem Netz aufliegt, und wobei das Netz ( 5 ) und die Membran ( 3, 30 ) durch geeignete Haltevorrichtungen ( 6 ), vorzugsweise nichleitende Ringe aus elastischem Material, am Elektrodenkörper festgehalten werden.

Das nachfolgende Beispiel soll die Erfindung näher erläutern, ohne sie darauf zu beschränken.


Beispiel

EP 0 394 997 A1

Bestimmung der Konzentration von Maltose in einer wäßrigen Maltoselösung.

Es wird mit einem Biosensor gemäß Figur 1b gearbeitet. Als Sensorelektrode (10) dient eine übliche Ag/AgCl-Elektrode. Der Abstandhalter (5) ist ein grobmaschiges Nylonnetz. Abstandshalter (5) und Membran (30) werden mit zwei Hartgummiringen (6) an den Elektrodenkörper (10) gepreßt. Als Elektrolyt wird eine 0.1 NaCl-Lösung verwendet, und als Referenzelektrode eine Ag/AgCl-Elektrode (salt bridge).

Als Membran dient eine Maltoporin aus E. coli enthaltene Membran mit einer PVC-Matrix. Das Maltoporin wurde wie bei B. Dargent et al, FEBS Let. 220 (1987) 136-142, beschrieben erhalten. Es wird einem üblichen Ansatz zur Herstellung von Polyvinylchlorid (durch Dispersionspolymerisation) zugegeben. Das fertige Polymere wird zu Filmen vergossen. In der fertigen Membran beträgt das Verhältnis von Maltoporin/Polyvinylchlorid $10^{-3}$.

Als Probelösungen werden Lösungen von Maltose mit einem Gehalt von $10^{-2}$, $5 \times 10^{-3}$, $10^{-3}$ und $5 \times 10^{-4}$ M Maltose/Liter im Elektrolyten verwendet.

Die Bestimmung der Differenz der Leitfähigkeiten zwischen Sensorelektrode und Referenzelektrode (1) unter Verwendung einer Lösung ohne Maltose, und (2) unter Verwendung der vier Probelösungen ergab in einer graphischen Darstellung von $G_{max}/(G_{max}-G_c)$ als Funktion von 1/C eine Gerade (die Abweichungen der Meßpunkte von der Geraden betrugen maximal ± 3 %).

## Ansprüche

1. Verwendung von Porinen gram negativer Bakterien für biospezifische Membranen von Biosensoren.

2. Verwendung nach Anspruch 1, dadurch **gekennzeichnet**, daß die biospezifische Membran aus einer Matrix, in der die Porine eingelagert sind, besteht.

3. Verwendung nach Anspruch 2, dadurch **gekennzeichnet**, daß die Matrix aus Lipiden oder gegebenenfalls vernetzten synthetischen Polymeren gebildet wird.

4. Verwendung nach einem der Ansprüche 1 bis 2, dadurch **gekennzeichnet**, daß das Porin ausgewählt ist aus der Gruppe LamB, PhoE und Ts$^x$.

5. Verwendung einer Membran aus einer Matrix mit darin eingelagerten Porinen gram negativer Bakterien als biospezi fische Membran von Biosensoren.

6. Verwendung nach Anspruch 5, dadurch **gekennzeichnet**, daß die Matrix aus Lipiden oder gegebenenfalls vernetzten synthetischen Polymeren gebildet wird.

7. Verwendung nach Anspruch 5 oder 6, dadurch **gekennzeichnet**, daß das Porin LamB, PhoE oder Ts$^x$ ist.

8. Biospezifische Membran zur Verwendung als sensitive Membran in Biosensoren, bestehend aus einer Matrix aus einem gegebenenfalls vernetzten synthetischen Polymeren mit darin eingelagerten Porinen gram negativer Bakterien.

9. Biospezifische Membran nach Anspruch 8, dadurch **gekennzeichnet**, daß sie als Porin LamB, PhoE oder Ts$^x$ enthält.

10. Verfahren zur Bestimmung von organischen Substanzen durch Messung der durch diese Substanzen hervorgerufenen Veränderung der Ionendurchlässigkeit einer Membran, dadurch **gekennzeichnet**, daß man als Membran eine ein für die zu bestimmende organische Substanz spezifisches Porin aus gram negativen Bakterien enthaltende Membran einsetzt und die durch die zu bestimmende organische Substanz hervorgerufene Veränderung der Durchlässigkeit von Ionen, die unter der Einwirkung eines elektrischen Feldes durch die Membran hindurchtreten, bestimmt.

11. Verfahren nach Anspruch 10, dadurch **gekennzeichnet**, daß man die Veränderung der Durchlässigkeit für die Ionen als Differenz der Leitfähigkeiten durch die Membran vor und nach ihrem Kontakt mit den zu bestimmenden organischen Substanzen mißt.

12. Verfahren nach Anspruch 10 oder 11, dadurch **gekennzeichnet**, daß man die Differenzbestimmung unter Verwendung der für Biosensoren üblichen Meßprinzipien durchführt, wobei man in diesen Biosensoren als biosensitive Membran eine Membran aus einer Matrix mit darin eingelagerten Porinen gram negativer Bakterien verwendet.

13. Verfahren nach Anspruch 12, dadurch **gekennzeichnet**, daß man als biosensitive Membran eine Membran nach einem der Ansprüche 8 oder 9 verwendet.

14. Verfahren nach einem der Ansprüche 10 bis 12, dadurch **gekennzeichnet**, daß man als Porin LamB, PhoE oder Ts$^x$ verwendet.

15. Biosensor, bestehend aus einer Sensorelektrode (1, 10), die zumindest teilweise über eine semipermeable Membran (3, 30) in Verbindung mit der zu untersuchenden Probelösung steht, dadurch **gekennzeichnet**, daß die semipermeable Membran (3, 30) eine Membran aus einer Matrix und darin eingelagerten Porinen gram negativer Bakterien ist.

16. Biosensor nach Anspruch 15, dadurch **gekennzeichnet**, daß die Elektrode (1, 10) stabförmig und an ihrem in die Probelösung eintauchenden Ende vorzugsweise abgerundet ausgebildet ist, wobei auf diesem Ende ein Netz (5) aus einem nicht-leitenden Material aufliegt, und die Membran (3, 30) auf diesem Netz aufliegt, und wobei das Netz (5) und die Membran (3, 30) durch geeignete Haltevorrichtungen (6), vorzugsweise nicht-leitende Ringe aus elastischem Material, am Elektrodenkörper festgehalten werden.

17. Biosensor nach Anspruch 15 oder 16, dadurch **gekennzeichnet**, daß die Membran (3, 30) ein Porin enthält ausgewählt aus der Gruppe LamB, PhoE und Ts$^x$.

18. Biosensor nach einem der Ansprüche 15 bis 17, dadurch **gekennzeichnet**, daß die Membran eine Matrix enthält, ausgewählt aus der Gruppe Lipide und gegebenenfalls vernetzte synthetische Polymere.

19. Biosensor nach Anspruch 18, dadurch **gekennzeichnet**, daß das synthetische Polymer Polyvinyl-chlorid (PVC) ist.

(a)

(b)

Fig. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN, vol. 13, no. 120 (P-846)[3468], 24th March 1989; & JP-A-63 292 057 (YAMAMOTO SEISAKUSHO K.K.) 29-11-1988 * Abstract * | 1-19 | G 01 N 27/30 C 12 M 1/40 |
| X | PATENT ABSTRACTS OF JAPAN, vol. 12, no. 451 (P-791)[3298], 28th November 1988; & JP-A-63 173 948 (TADASHI MATSUNAGA) 18-07-1988 * Whole abstract * | 1,4,10, 14,15, 17 | |

**TECHNICAL FIELDS SEARCHED (Int. Cl.5)**

C 12 M
G 01 N

The present search report has been drawn up for all claims

| Place of search | Date of completion of the search | Examiner |
|---|---|---|
| THE HAGUE | 26-06-1990 | EPAILLARD P.J.H.M. |